# EUROPEAN PATENT SPECIFICATION

(11) **EP 2 129 377 B1**
(45) Date of publication and mention of the grant of the patent: **05.10.2011**
(21) Application number: 08715616.2
(22) Date of filing: 14.03.2008
(51) Int. Cl.: A61K 31/451, A61P 25/00, A61K 9/08

(54) **LIQUID FORMULATIONS OF SALTS OF 4-[2-(4-METHYLPHENYLSULFANYL)PHENYL]PIPERIDINE**
FLÜSSIGE FORMULIERUNGEN VON SALZEN VON 4-[2-(4-METHYLPHENYLSULFANYL)PHENYL]PIPERIDIN
FORMULATIONS LIQUIDES DE SELS DE 4-[2-(4-MÉTHYLPHÉNYLSULFANYL)PHÉNYL]PIPÉRIDINE

(30) Priority: 20.03.2007 DK 200700423; 15.06.2007 WO PCT/DK2007/050076; 14.12.2007 DK 200701790; 14.12.2007 US 13918
(43) Date of publication of application: 09.12.2009
(60) Divisional application: 11177654.8
(73) Proprietor: H. Lundbeck A/S, 2500 Valby-Copenhagen (DK)
(72) Inventor: LASSKOGEN, Gudrun, DK-2650 Hvidovre (DK); STENSBØL, Tine, Bryan, DK-3500 Værløse (DK); LOPEZ DE DIEGO, Heidi, DK-2850 Nærum (DK)
(74) Representative: Conrad, Lars Sparre
(86) International application number: PCT/DK2008/050062
(87) International publication number: WO 2008/113358

(56) References cited:
- WO-A-03/029232
- WO-A-2007/144006
- GUTIERREZ MARCELO M ET AL: "Pharmacokinetic comparison of oral solution and tablet formulations of citalopram: A single-dose, randomized, crossover study" CLINICAL THERAPEUTICS, EXCERPTA MEDICA, PRINCETON, NJ, US, vol. 22, no. 12, 1 December 2000 (2000-12-01), pages 1525-1532, XP002424856 ISSN: 0149-2918

## Description

### Field of the invention

The present invention relates to liquid pharmaceutical composition.

### Background of the invention

The international patent application published as WO 2003/029232 discloses e.g. the compound 4-[2-(4-methylphenylsulfanyl)phenyl]piperidine (compound I) as a free base and the corresponding HCl addition salt. The compound is reported to be an inhibitor of the serotonin transporter and the serotonin receptor 2C (5-HT_{2C}), and is said to be useful for the treatment of affective disorders, e.g. depression and anxiety.

It has been found that compound I is also a potent antagonist of the serotonin receptor 3 (5-HT₃) and the serotonin receptor 2A (5-HT_{2A}) wherefore compound I may be used for the treatment of a broader range of indication including pain and cognitive impairment. This pharmacological profile was also disclosed in WO 2007/144006.

For many pharmaceutical compounds oral administration of a tablet, capsule, pill or similar intended for swallowing is the preferred administration form. However, some patients, e.g. elderly patients may have difficulties swallowing, and liquid solutions may be a suitable alternative avoiding the need for swallowing tablets, capsules, pills, etc. A liquid solution further provides a possibility of a flexible dosing regime. In order to limit the volume of a liquid solution it is necessary to have a high concentration of the active ingredient in the solution, which again requires a high solubility of the active ingredient.

The present invention is related to liquid formulations of compound I.

### Summary of the invention

The present inventors have surprisingly found that the DL-lactic acid addition salt (=DL-lactate), the glutaric acid addition salt (=gluterate), the L-aspartic acid addition salt (=L-aspartate) and the glutamic acid addition salt (=glutamate) of 4-[2-(4-methylphenylsulfanyl)phenyl]piperidine are exceptionally soluble. Accordingly, the present invention relates to a liquid formulation comprising the DL-lactic acid addition salt, the glutaric acid addition salt, the L-aspartic acid addition salt or the glutamic acid addition salt of 4-[2-(4-methylphenylsulfanyl)phenyl]piperidine.

In one embodiment, the invention relates to the use of salts of the present invention in the manufacture of a liquid pharmaceutical composition for the treatment of certain diseases.

In one embodiment, the invention relates to salts of the present invention for use in the treatment of certain diseases, wherein said salts are in a liquid formulation.

In one embodiment, the present invention relates to a container comprising a liquid formulation of the present invention, wherein said container is fitted with a drop aggregate.

### Figures

Figure 1: Acetylcholine levels in the prefrontal cortex and ventral hippocampus upon administration of compound I
Figure 2: Dopamine levels in prefrontal cortex upon administration of compound I

### Detailed description of the invention

The formulation to which the present invention relates are all pharmaceutical formulations.

Table 2 in the examples shows the solubility of various salts of 4-[2-(4-methylphenylsulfanyl)phenyl]piperidine. As evidenced by the data, the DL-lactic acid, the L-aspartic acid, the glutamic acid, and the glutaric acid addition salts have exceptionally high solubility. For convenience, these salts are referred to as the salts of the present invention.

DL lactic acid is also known as DL-2-hydroxypropionic acid, and it forms and 1:1 acid addition salt with 4-[2-(4-methylphenylsulfanyl)phenyl]piperidine which is used in the present invention.

Glutaric acid is also known as 1,5-pentanedioic acid, and it forms a 1:1 acid addition salt with 4-[2-(4-methylphenylsulfanyl)phenyl]piperidine which is used in the present invention.

L-aspartic acid forms a 1:1 acid addition salt with 4-[2-(4-methylphenylsulfanyl)phenyl]piperidine which is used in the present invention.

Glutamic acid forms a 1:1 acid addition salt with 4-[2-(4-methylphenylsulfanyl)phenyl]piperidine which is used in the present invention.

4-[2-(4-methylphenylsulfanyl)phenyl]piperidine may be prepared as disclosed in WO 2003/029232. Alternatively, 4-[2-(4-methylphenylsulfanyl)phenyl]piperidine may be prepared as described in the examples. The salts of the present invention may be prepared by addition of the appropriate acid followed by precipitation, which precipitation may be brought about by e.g. cooling, removal of solvent, addition of another solvent or a mixture thereof. The examples disclose specific routes for obtaining the salts.

Liquid formulations may be intended for oral or parenteral administration. Liquid formulations for parenteral administration, which include infusion solutions, are in many aspects similar to other liquid formulations, but are additionally characterised by being sterile and isotonic.

The liquid oral formulation of the present invention may be presented as a syrup, an elixir, an oral solution, a suspension, or as a concentrated oral formulation. One advantage of these administration forms is that the patient does not have to swallow a solid form, which may be difficult, in particular for elderly patients or for patients with traumas in the mouth or throat.

Syrups and elixirs are typically sweetened, flavoured liquids containing an active pharmaceutical ingredient. Syrups typically have a higher sugar content, and elixirs often contain alcohol as well. An oral solution is a solution of the active ingredient. A suspension is a two-phase system comprising solid particles dispersed in a liquid. Administration of syrups, elixirs, oral solutions and suspensions typically involves the intake of relatively large amounts of liquid, i.e. 10-50 ml.

In contrast hereto, the concentrated oral formulations of the present invention are administered to the patient by measuring out a pre-determined volume of said formulation from a suitable dispenser, adding the resulting volume to a glass of liquid (beverage, e.g. water, juice or similar) upon which the patient drinks the liquid. For convenience, the volume measured out is small, e.g. less than 2 ml, such as less than 1 ml, such as less than 0.5 ml. In a particular embodiment, the concentrated oral formulations of the present invention are administered to the patient by measuring out a pre-determined number of drops of said formulation from a suitable dispenser, e.g. a container with a drop aggregate, adding the drops to a glass of liquid (water, juice or similar) upon which the patient drinks the liquid. In this context, a drop aggregate is an aggregate fitted to a container that effects that a liquid inside said container may be dispensed from said container in discrete drops.

The concentration of the salts of the present invention in concentrated oral formulations is determined by the number of drops (or the volume) it is desired to collect and the amount of the salts it is desired to administer. It is generally held that measuring out around 10-20 drops is an optimal compromise between safety/efficacy of the treatment on the one hand and convenience on the other. If the concentration of the salts of the present invention is too high, i.e. if only a low number of drops is to be measured out, it may jeopardize safety or efficacy of the treatment. With a low number of drops, one or two drops more or less than desired will significantly increase the uncertainty in the dose provided. On the other hand, if the concentration of the salts of the present invention is too low, the number of drops to be measured out is high, which is inconvenient for the patient or the caretaker.

With daily dosages of salts of the present invention of 5 mg, a concentrated oral formulation with a concentration of 5 mg of active ingredient per ml could be appropriate. A concentration of 5 mg per ml and a drop number of 20 drops/ml would make it possible to administer 20 drops for a dose of 5 mg.

With daily dosages of salts of the present invention of 5 mg, a concentrated oral formulation with a concentration of 10 mg of active ingredient per ml could be appropriate. A concentration of 10 mg per ml and a drop number of 20 drops/ml would make it possible to administer 10 drops for a dose of 5 mg.

With daily dosages of compounds of the present invention of 10 mg, a concentrated oral formulation with a concentration of 20 mg of active ingredient per ml could be appropriate. A concentration of 20 mg per ml and a drop number of 20 drops/ml would make it possible to administer 10 drops for a dose of 10 mg.

With daily dosages of compounds of the present invention of 20 mg, a concentrated oral formulation with a concentration of 40 mg of active ingredient per ml could be appropriate. A concentration of 40 mg per ml and a drop number of 20 drops/ml would make it possible to administer 10 drops for a dose of 20 mg.

With daily dosages of compounds of the present invention of 30 mg, a concentrated oral formulation with a concentration of 60 mg of active ingredient per ml could be appropriate. A concentration of 60 mg per ml and a drop number of 20 drops/ml would make it possible to administer 10 drops for a dose of 30 mg.

With daily dosages of compounds of the present invention of 40 mg, a concentrated oral formulation with a concentration of 80 mg of active ingredient per ml could be appropriate. A concentration of 80 mg per ml and a drop number of 20 drops/ml would make it possible to administer 10 drops for a dose of 40 mg.

With daily dosages of compounds of the present invention of 50 mg, a concentrated oral formulation with a concentration of 100 mg of active ingredient per ml could be appropriate. A concentration of 100 mg per ml and a drop number of 20 drops/ml would make it possible to administer 10 drops for a dose of 50 mg.

In one embodiment, the concentrated oral drop formulation is administered from an apparatus with a pen or syringe like dosing device. Examples of such administering apparatus are provided in e.g. WO 03/061508, US 2004/0186431, US 2003-089743. These apparatuses contain the concentrated oral formulation of the present invention in a sealed chamber, e.g. an a cartridge and they have a means for providing a metered amount of the concentrated oral solution. In order to limit the size of the device, the volume of the concentrated oral solution is small (0.01-1 ml) which again requires the salts to be highly soluble. These pen or syringe like dosing devices are convenient for the patient in that that they may be carried e.g. in a breast pocket, and a suitable design of the device may serve to hide that it is a dispenser of a drug.

With daily dosages of salts of the present invention of 5 to 10 mg, a concentrated oral formulation with a concentration of 100 mg active ingredient per ml would be appropriate. This would mean that 0.05 ml is delivered for a dose of 5 mg and that 0.1 ml is delivered for a dose of 10 mg.

With daily dosages of compounds of the present invention of 20-50 mg, a concentrated oral formulation with a concentration of 200 mg active ingredient per ml would be appropriate. This would mean that 0.1 ml is delivered for a dose of 20 mg and that 0.25 ml is delivered for a dose of 50 mg.

Accordingly, concentrated oral formulations of the present invention comprise approximately 5-250 mg/ml of salts of the present invention. Particular examples include approximately 10-100 mg/ml, approximately 20-200 mg/ml, approximately 150-200 mg/ml, approximately 20-80 mg/ml, approximately 30-70 mg/ml, and approximately 10, 20, 30, 40, 50, 60, 70, 80, 90 or 100 mg/ml. In one embodiment, the oral drop formulation of the present invention comprise at least 5 mg/ml of salts of the present invention. In one embodiment, the oral drop formulation of the present invention comprise at least 10 mg/ml of salts of the present invention. In one embodiment, the oral drop formulation of the present comprises at least 20 mg/ml of a salt of the present invention. In one embodiment, the oral drop formulation of the present comprises at least 30 mg/ml of a salt of the present invention. In one embodiment, the oral drop formulation of the present comprises at least 50 mg/ml of a salt of the present invention. In one embodiment, the oral drop formulation of the present comprises at least 80 mg/ml of a salt of the present invention

In addition to the salts of the present application, the oral solutions of the present application, and in particular the concentrated oral formulations may comprise solvents, buffers, surfactants, surface tension modifiers, viscosity modifiers, preservatives, antioxidants, colorants, taste maskers, flavours etc.

Examples of solvents include water and other solvents, which are miscible with water or solubilizing agents, and are suitable for oral purposes. Examples of suitable solvents are ethanol, propylene glycol, glycerol, polyethylene glycols, poloxamers, sorbitol, benzyl alcohol. The aqueous solubility of the active ingredient may further be enhanced by the addition to the solution of a pharmaceutically acceptable co-solvent, a cyclodextrin or a derivative thereof.

A buffer system may be used to maintain the pH of the formulation in an optimal pH-range. A buffer system is a mixture of appropriate amounts of a weak acid such as acetic, phosphoric, succinic, tartaric, lactic or citric acid and its conjugate base. Ideally, the buffer system has sufficient capacity to remain in the intended pH range upon dilution with a neutral, a slightly acidic or a slightly basic beverage.

Surfactants are substances, which solubilize active compounds, which are insufficiently soluble in an aqueous medium, usually with the formation of micelles. Preferably the surfactant used should be non-ionic due to less toxicity. High concentrations of surfactants may be used to allow for dilution during administration without precipitation. Examples of surfactants include tweens, spans and mono- and diglycerides. Surface tension modifiers may be included to adjust the drop number for the oral drop formulations. An example of surface tension modifier is ethanol, which decreases the surface tension and increases the drop number.

Viscosity modifiers may be included to adjust the drop velocity for a concentrated oral formulation. The drop velocity for a formulation to be measured out in discrete drops from a container fitted with a drop aggregate should for convenience not exceed 2 drops per second. Examples of viscosity modifiers include ethanol, hydroxyethylcellulose, carboxymethylcellulose sodium, methylcellulose, polyvinyl alcohol, polyvinylpyrrolidone, polyethylene glycol and glycerine.

Preservative agents may be added to prevent the growth of microorganisms such as bacteria, yeasts and fungi in liquid formulations, which are likely to be used repeatedly. Suitable preservatives should be pharmaceutically acceptable, physicochemical stable and effective in the desired pH range. Examples of preservative agents include ethanol, benzoic acid, sorbic acid, methylparaben, propylparaben and benzyl alcohol.

Since a drug substance is more sensitive to chemical degradation in dissolved than in solid form, it may be necessary to include an antioxidant in the liquid formulation. Examples of antioxidants include propyl gallate, ascorbyl palmitate, ascorbic acid, sodium sulphite, citric acid and EDTA.

Colouring agents may be used in some formulations to introduce a uniformity of appearance to the product. Some active ingredients may further be very sensitive to light and it may prove necessary to add colouring agents to the drop formulations to protect them from light and for the purpose of stabilization. Suitable colouring agents include for example tartrazine and sunset yellow.

Sweetening agents may mask unpleasant taste associated with some formulations or to achieve a desired taste. Examples of sweetening agents are saccharin, sodium salt of saccharin, glucose, sorbitol, glycerol, acesulfame potassium and neohesperidin dihydrochalcon. The taste may be optimized further by the addition of one or more flavouring substances. Suitable flavouring substances are fruit flavours such as cherry, raspberry, black currant, lemon or strawberry flavour or other flavours such as liquorish, anis, peppermint, caramel etc.

Particular examples of a concentrated oral formulation which may be administered with a drop aggregate are (4-[2-(4-methylphenylsulfanyl)-phenyl]piperidine = Compound I)
0.73% Compound I glutarate corresponding to 0.5% Compound I free base Water q.s. ad 100%
1.58% Compound I glutamate corresponding to 1% Compound I free base 0.08% methylparahydroxybenzoate
0.02% propylparahydroxybenzoate
Water q.s. ad 100%
2.94% Compound I L-aspartate corresponding to 2% Compound I free base 0.08% methylparahydroxybenzoate
0.02% propylparahydroxybenzoate
7% ethanol
Water q.s. ad 100%
10.54% Compound I DL-lactate corresponding to 8% Compound I free base 0.08% methylparahydroxybenzoate
0.02% propylparahydroxybenzoate
Water q.s. ad 100%

A particular example of a concentrated oral formulation, which may be administered with a pen or syringe like device, is
26.36% Compound I DL-lactate corresponding to 20% Compound I free base 0.08% methylparahydroxybenzoate
0.02% propylparahydroxybenzoate
Water q.s. ad 100%

The pharmaceutical profile of the salts of the present invention is disclosed in the examples. In summary, the salts of the present invention are inhibitors of the serotonin transporter and the serotonin receptor 2C (5-HT_{2C}) and antagonists of the serotonin receptor 3 (5-HT₃), the serotonin receptor 2A (5-HT_{2A}) and the α₁ adrenergic receptor and they appear to bring about an increase in the extracellular level of acetylcholine in the prefrontal cortex and ventral hippocampus and the dopamine level in the prefrontal cortex. Additionally, the salts of the present invention are effective in the treatment of pain as shown in the examples. The pharmacological profile in combination with the pre-clinical data are expected to be reflected in the clinical utility of the salts of the present invention. Hence, the salts of the present invention are believed to be useful in the treatment of diseases including mood disorders, major depressive disorder, general anxiety disorder, atypical depression, bipolar depression, social anxiety disorder, obsessive compulsive disorder, panic disorder, post traumatic stress disorder, abuse, eating disorder, sleep disorder, Alzheimer's disease, dementia, chronic pain, depression associated with cognitive impairment, depression associated with psychosis, cognitive impairment in schizophrenia, depression or anxiety associated with pain, behavioural disturbances in the elderly, ADHD, melancholia treatment resistant depression and depression with residual symptoms.

5-HT_{2C} receptors are located e.g. on dopaminergic neurons where activation exerts a tonic inhibitory influence on the dopamine release, and 5-HT_{2C} antagonists will effect an increase in the dopamine level. Data presented in the examples show that compound I does, in deed, bring about an increase in the extra cellular dopamine levels in the brain. On this background it may be hypothesized that 5-HT_{2C} antagonists are particular well-suited for the treatment of depression which is refractory to the treatment with selective serotonin reuptake inhibitors. This hypothesis finds support in several clinical studies showing a combination of mirtazipine and SSRI to be superior to SSRI alone for the treatment of depressed patients with an inadequate clinical response (treatment resistant depression, TRD, or refractory depression) [Psychother. Psychosom., 75, 139-153, 2006]. Mirtazapine is also a 5-HT₂ and a 5-HT₃ antagonist, which indicate that compounds exerting serotonin reuptake inhibition in combination with 5-HT₂ and 5-HT₃ antagonism, such as compound I, are useful for the treatment of TRD, i.e. will increase the remission rate for patients suffering from treatment resistant depression.

Data presented in the examples shows that compound I brings about an increase in the extracellular level of acetylcholine in the brain. There is longstanding clinical evidence that increasing the acetylcholine levels in the brain is a way to treat Alzheimer's and cognitive impairment in general, cf. the use of acetylcholine esterase inhibitors in the treatment of Alzheimer's. On this background, compound I is believed to be useful in the treatment of Alzheimer's and cognitive impairment, and also mood disorders, such as depression associated with Alzheimer's and cognitive impairment.

A segment of depressed patients will respond to treatment with e.g. SSRI in the sense that they will improve on clinically relevant depression scales, such as MADRD and HAMD, but where other symptoms, such as sleep disturbances and cognitive impairment remain. In the present context, these patients are referred to as partial responders and as suffering from depression with residual symptoms. Due to the above discussed effects on the acetylcholine levels, compound I is expected to be useful in the treatment of the cognitive impairment in addition to the depression. Clinical studies have shown that the compound prazosin, which is an α-1 adrenergic receptor antagonist reduces sleep disturbances [Raskind, Biol. Psychiatry, 2006 in press]. Moreover, the 5-HT_{2A} and 5-HT_{2C} antagonism of the compounds of the present invention is also believed to have a sedative, sleep-improving effect [Neuropharmacol, 33, 467-471, 1994] wherefore the compounds of the present invention are useful for the treatment of partial responders (depression with residual symptoms), or rephrased that treatment of depressed patients with compound I will reduce the fraction of partial responders.

The compound of the invention may be administered in an amount of about 0.001 to about 100 mg/kg body weight per day.

A typical oral dosage is in the range of from about 0.001 to about 100 mg/kg body weight per day, preferably from about 0.01 to about 50 mg/kg body weight per day, administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

A typical oral dosage for adults is in the range of 1-100 mg/day of a compound of the present invention, such as 1-30 mg/day, or 5-25 mg/day. This may typically be achieved by the administration of 0.1-50 mg, such as 1-25 mg, such as 1, 5, 10, 15, 20 25, 30, 40, 50 or 60 mg of the compound of the present invention once or twice daily.

A "therapeutically effective amount" of a compound as used herein means an amount sufficient to cure, alleviate or partially arrest the clinical manifestations of a given disease and its complications in a therapeutic intervention comprising the administration of said compound. An amount adequate to accomplish this is defined as "therapeutically effective amount". The term also includes amounts sufficient to cure, alleviate or partially arrest the clinical manifestations of a given disease and its complications in a treatment comprising the administration of said compound. Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician.

The term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. Nonetheless, prophylactic (preventive) and therapeutic (curative) treatment are two separate aspect of the invention. The patient to be treated is preferably a mammal, in particular a human being.

In one embodiment, the invention relates to the use of a salt of the present invention for the manufacture of a liquid formulation for the treatment of a disease selected from mood disorders, major depressive disorder, general anxiety disorder, atypical depression, bipolar depression, social anxiety disorder, obsessive compulsive disorder, panic disorder, post traumatic stress disorder, abuse, eating disorder, sleep disorder, Alzheimer's disease, dementia, chronic pain, depression associated with cognitive impairment, depression associated with psychosis, cognitive impairment in schizophrenia, depression or anxiety associated with pain, behavioural disturbances in the elderly, ADHD, melancholia, treatment resistant depression or depression with residual symptoms. In one embodiment, said liquid formulation is a concentrated oral formulation.

In one embodiment, the present invention relates to salts of the present invention for use in the treatment of a disease selected from mood disorders, major depressive disorder, general anxiety disorder, atypical depression, bipolar depression, social anxiety disorder, obsessive compulsive disorder, panic disorder, post traumatic stress disorder, abuse, eating disorder, sleep disorder, Alzheimer's disease, dementia, chronic pain, depression associated with cognitive impairment, depression associated with psychosis, cognitive impairment in schizophrenia, depression or anxiety associated with pain, behavioural disturbances in the elderly, ADHD, melancholia, treatment resistant depression or depression with residual symptoms, wherein said salt is in a liquid formulation. In one embodiment, said liquid formulation is a concentrated oral drop formulation.

The salts of the present invention may either be administered alone or in combination with another therapeutically active compound, wherein the two compounds may either be administered simultaneously or sequentially. Examples of therapeutically active compounds which may advantageously be combined with compound I include sedatives or hypnotics, such as benzodiazepines; anticonvulsants, such as lamotrigine, valproic acid, topiramate, gabapentin, carbamazepine; mood stabilizers such as lithium; dopaminergic drugs, such as dopamine agonists and L-Dopa; drugs to treat ADHD, such as atomoxetine; psychostimulants, such as modafinil, ketamine, methylphenidate and amphetamine; other antidepressants, such as mirtazapine, mianserin and buproprion; hormones, such as T3, estrogen, DHEA and testosterone; atypical antipsychotics, such as olanzapine and aripiprazole; typical antipsychotics, such as haloperidol; drugs to treat Alzheimer's diseases, such as cholinesterase inhibitors and memantine, folate; S-Adenosyl-Methionine; immunmodulators, such as interferons; opiates, such as buprenorphins; angiotensin II receptor 1 antagonists (AT1 antagonists); ACE inhibitors; statins; and alphal1 adrenergic antagonist , such as prazosin.

The use of the terms "a" and "an" and "the" and similar referents in the context of describing the invention are to be construed to cover both the singular and the plural, unless otherwise indicated herein or clearly contradicted by context. For example, the phrase "the compound" is to be understood as referring to various "compounds" of the invention or particular described aspect, unless otherwise indicated.

Unless otherwise indicated, all exact values provided herein are representative of corresponding approximate values (e.g., all exact exemplary values provided with respect to a particular factor or measurement can be considered to also provide a corresponding approximate measurement, modified by "about," where appropriate).

The description herein of any aspect or aspect of the invention using terms such as "comprising", "having," "including," or "containing" with reference to an element or elements is intended to provide support for a similar aspect or aspect of the invention that "consists of", "consists essentially or, or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

### Examples

### Example 1 Pharmacological profile

### Example 1A Serotonin (5-HT) and norepinephrine (NE) reuptake inhibition

Aliquots of test compound and rat cortical synaptosome preparation were preincubated for 10 min/37°C, and then added [³H]NE or [³H]5-HT (final concentration 10 nM). Non-specific uptake was determined in the presence of 10µM talsupram or citalopram and the total uptake was determined in the presence of buffer. Aliquots were incubated for 15 minutes at 37 °C. After the incubation [³H]NE or [³H]5-HT taken up by synaptosomes was separated by filtration through Unifilter GF/C, presoaked in 0.1 % PEI for 30 minutes, using a Tomtec Cell Harvester program. Filters were washed and counted in a Wallac MicroBeta counter.

At NET compound I displays an IC₅₀ value of 23 nM. At SERT compound I displays an IC₅₀ value of 8 nM.

### Example 1B 5-HT_{2A} antagonism

Compound I was tested for affinities towards serotonin receptors and was found to exhibit an antagonistic profile with affinity at 5-HT_{2A} receptors (Kᵢ 54 nM). The affinity is calculated from Y = 100/(1+ 10^{(X-logIC}₅₀⁾) where Y denotes % binding and X denotes the concentration of compound. 5 concentrations of compound (1,10, 30, 100, 1000 nM) were used to calculate the IC₅₀ value. Kᵢ was calculated from the Cheng Prusoff equation Kᵢ = (IC₅₀/(1+ ([L]/Kd)) Affitiny was determined at MDL Pharmaservices catalogue number 271650.

In mammalian cells expressing human 5-HT_{2A} receptors compound I displays competitive antagonistic properties. The compound binds to 5-HT_{2A} receptors with a Ki of < 100 nM and in a functional assay the compounds antagonise 5-HT evoked release of Ca²⁺ from intracellular stores with a Kb of 67 nM. A schild analysis revealed competitive antagonism with a Kb of 100 nM.

The experiment was carried out as follows. 2 or 3 days before the experiment CHO cells expressing 250 fmol/mg human 5-HT_{2A} receptors are plated at a density sufficient to yield a mono-confluent layer on the day of the experiment. The cells are dye loaded (Ca²⁺-kit from Molecular Devices) for 60 minutes at 37° C in a 5% CO₂ incubator at 95% humidity. Basal fluorescence was monitored in a fluorometric imaging plate reader or FLIPR³⁸⁴ from Molecular Devices (Sunnyvale, CA) with an excitation wavelength of 488 nm and an emission range of 500 to 560 nm. Lacer intensity was set to a suitable level to obtain basal values of approximately 8000-10000 fluorescence units. The variation in basal fluorescence should be less than 10%. EC₅₀ values are assessed using increasing concentrations of test compound covering at least 3 decades. pA2 values are assessed challenging full dose response curves of 5-HT with four different concentrations of compound (150, 400 1500 and 4000 nM). Kb values were also assessed challenging 2 decades of concentrations of test substances with EC₈₅ of 5-HT. Test substances are added to the cells 5 minutes before the 5-HT. Kᵢ values were calculated using Cheng-Prusoff equation.

### Example 1C 5-HT_{3A} receptor antagonism

In oocytes expressing human-homomeric 5-HT_{3A} receptors 5-HT activates currents with an EC₅₀ of 2600 nM. This current can be antagonised with classical 5-HT₃ antagonists such as ondansetron. Ondansetron displays a Kᵢ value below 1 nM in this system. Compound I exhibits potent antagonism in low concentrations (0.1 nM - 100 nM) (IC₅₀ ~ 10 nM/ Kb ~ 2 nM) and agonistic properties when applied in higher concentrations (100 - 100000 nM) (EC₅₀ ~ 2600 nM) reaching a maximal current of approximately 70-80 % of the maximal current elicited by 5-HT itself. In oocytes expressing rat-homomeric 5-HT_{3A} receptors 5-HT activates currents with an EC₅₀ of 3.3 µM. The experiments were carried out as follows. Oocytes were surgically removed from mature female *Xenepus laevis* anaesthetized in 0.4 % MS-222 for 10 - 15 min. The oocytes were then digested at room temperature for 2-3 hours with 0.5 mg/ml collagenase (type IA Sigma-Aldrich) in OR2 buffer (82.5 mN NaCl, 2.0 mM KCl, 1.0 mM MgC12 and 5.0 mM HEPES, pH 7.6). Oocytes avoid of the follicle layer were selected and incubated for 24 hours in Modified Barth's Saline buffer [88 mM NaCl, 1 mM KCl, 15 mM HEPES, 2.4 mM NaHCO₃, 0.41 mM CaCl₂, 0.82 mM MgSO₄, 0.3 mM Ca(NO₃)₂] supplemented with 2 mM sodium pyruvate, 0.1 U/l penicillin and 0.1 µg/l streptomycin. Stage IV-IV oocytes were identified and injected with 12-48 nl of nuclease free water containing 14 - 50 pg of cRNA coding for human 5-HT3A receptors receptors and incubated at 18°C until they were used for electrophysio logical recordings (1 - 7 days after injection). Oocytes with expression of human 5-HT3 receptors were placed in a 1 ml bath and perfused with Ringer buffer (115 mM NaCl, 2.5 mM KCl, 10 mM HEPES, 1.8 mM CaCl₂, 0.1 mM MgCl₂, pH 7.5). Cells were impaled with agar plugged 0.5-1 MΩ electrodes containing 3 M KCl and voltage clamped at -90 mV by a GeneClamp 500B amplifier. The oocytes were continuously perfused with Ringer buffer and the drugs were applied in the perfusate. 5-HT agonist-solutions were applied for 10 - 30 sec. The potencies of 5-HT₃ receptor antagonists were examined by measuring concentration-response against 10 µM 5-HT stimulation.

### Example 1D α_{1A} receptor antagonism

Compound I was tested for affinities towards the α_{1A} receptor and was found to exhibit an antagonistic profile with medium affinity for α_{1A} receptors (Ki = 34 nM).

On the day of the experiments membranes (see below for description of membrane preparation) are thawed and homogenized in buffer using an ultra turrax and diluted to the desired concentration (5 µg / well ~ 5 µg / 900 µl, store on ice until use).

The experiment is initiated by mixing of 50 µl test compound, 50µl [³H]-Prazosin and 900µl membranes, and the mixture is incubated for 20 minutes at 25 °C. Non-specific binding is determined in the presence of 10µM WB-4101 and the total binding is determined in the presence of buffer. After the incubation, bound ligand is separated from unbound by filtration through Unifilter GF/B, presoaked in 0.1 % PEI for 30 minutes, using a Tomtec Cell Harvester program (D4.2..4). 96 well. Filters are washed 3 times with 1 ml ice-cold buffer, dried at 50 °C and 35µl scintillation liquid/well is added to the filters. Bound radioactivity is counted in a Wallac OY 1450 MicroBeta. The affinity is calculated from Y = 100/(1+ 10(^{(X-logIC}₅₀⁾) where Y denotes % binding and X denotes the concentration of compound. Concentrations of compound covering 2 decades were used to calculate the IC50 value. Ki was calculated from the Cheng Prusoff equation Ki = (IC₅₀/(1+ ([L]/Kd))

In a functional assay compounds of the present invention antagonises adrenaline evoked release of Ca²⁺ from intracellular stores and a functional assay revealed that compounds were antagonists.

These experiments were carried out essentially as described below.

All cells were cultured in DMEM medium supplemented with 10% BCS, 4 mM L-glutamine (or 2 mM in the case of COS-7), and 100 units/ml penicillin plus 100 µg/ml streptomycin, at 37 oC, in 5% CO2.

Twenty-four hours prior to assays, CHO cells expressing the human alpha_{1A-7} receptors were seeded into 384-well black wall microtiter plates coated with poly-D-lysine. Culture medium was aspirated and cells were dye-loaded with 1.5 µM Fluo-4 in assay buffer composed of Hank's Balanced Salt Solution (138 mM NaCl, 5 mM KCl, 1.3 mM CaCl₂, 0.5 mM MgCl₂, 0.4 mM MgSO₄, 0.3 mM KH₂PO₄, 0.3 mM Na₂HPO₄, 5.6 mM glucose) plus 20 mM HEPES pH 7.4, 0.05% BSA and 2.5 mM probenicid (50 µl/well) for 1 hour in 5% CO₂ at 37 °C. After excess dye was discarded, cells were washed in assay buffer and layered with a final volume equal to 45 µl/well (or 30 ul/well for antagonist assay). In the case of antagonist evaluation, antagonist or vehicle was added at this point as a 15 µl aliquot in 4% DMSO-containing buffer at 4x the final concentration (final DMSO = 1%), followed by a 20 min incubation. Basal fluorescence was monitored in a fluorometric imaging plate reader or FLIPR^{™} from Molecular Devices (Sunnyvale, CA) with an excitation wavelength of 488 nm and an emission range of 500 to 560 nm. Laser excitation energy was adjusted so that basal fluorescence readings were approximately 8,000 relative fluorescent units (RFU). Cells were then stimulated at room temperature with agonists diluted in assay buffer (15 µl), and RFU were measured at 1.5 second intervals over a period of 2.5 min. Maximum change in fluorescence was calculated for each well. Concentration-response curves derived from the maximum change in fluorescence were analyzed by nonlinear regression (Hill equation). For antagonistic determinations, after 20 min of compound incubation (as above), fixed concentrations of standard agonist serotonin were added.

### Example 2A 4-[2-(4-methylphenylsulfanyl)phenyl]piperidine, HBr salt

### 2-(4-tolylsulfanyl)-phenyl bromide

In a stirred nitrogen covered reactor N-methyl-pyrrolidone, NMP (4.5L) was flushed with nitrogen for 20 minutes. 4-Methylbenzenethiol (900g, 7.25ml) was added and then 1,2-dibromobenzene (1709g, 7.25mol). Potassium tert-butoxide (813g, 7.25mol) was finally added as the last reactant. The reaction was exothermic giving a temperature rise of the reaction mixture to 70°C. The reaction mixture was then heated to 120°C for 2 - 3 hours. The reaction mixture was cooled to room temperature. Ethyl acetate (4L) was added and aqueous sodium chloride solution (15%, 2.5L). The mixture was stirred for 20 minutes. The aqueous phase was separated and extracted with another portion of ethyl acetate (2L). The aqueous phase was separated and the organic phases were combined and washed with sodium chloride solution (15%, 2.5L) The organic phase was separated, dried with sodium sulphate and evaporated at reduced pressure to a red oil which contains 20 - 30% NMP. The oil was diluted to twice the volume with methanol and the mixture was refluxed. More methanol was added until a clear red solution was obtained. The solution was cooled slowly to room temperature while seeded. The product crystallises as off white crystals, they were isolated by filtration and washed with methanol and dried at 40°C in a vacuum oven until constant weight.

### Ethyl 4-hydroxy-4-(2-(4-tolylsumanyl)phenyl)-peperidin-1-carboxylate

In a stirred reactor under nitrogen cover 2-(4-tolylsulfanyl)-phenyl bromide (600g, 2.15mol) was suspended in heptane (4.5L). At room temperature 10M BuLi in hexane (235mL, 2.36mol) was added over 10 minutes. Only a small exotherm was noticed. The suspension was stirred for 1 hour at ambient temperature and then cooled down to -40°C. 1-Carbethoxy-4-piperidone (368g, 2.15 mol) dissolved in THF (1.5L) was added at a rate not faster than the reaction temperature was kept below -40°C. When the reaction has gone to completion, it was warmed to 0°C and 1M HC1 (1L) was added keeping the temperature below 10°C. The acid aqueous phase was separated and extracted with ethyl acetate (1L). The organic phases were combined and extracted with sodium chloride solution (15%, 1L). The organic phase was dried over sodium sulphate and evaporated to a semi crystalline mass. It was slurried with ethyl ether (250 mL) and filtered off. Dried in an vacuum oven at 40°C until constant weight.

### Ethyl. 4-(2-(4-tolylsulfanyl)phenyl)-piperidin-1-carboxylate

Trifluoroacetic acid (2.8kg, 24.9mol) and triethylsilane (362g, 3.lmol) was charged in a reactor with an efficient stirrer. Ethyl 4-hydroxy-4-(2-(4-tolylsulfanyl)phenyl)-piperidin-1-carboxylate (462g, 1.24ml) was added via a powder funnel in portions. The reaction was slightly exothermic. The temperature rose to 50°C. After the addition was finalised the reaction mixture was warmed to 60°C for 18 hours. The reaction mixture was cooled down to room temperature. Toluene (750mL) and water (750mL) was added. The organic phase was isolated and the aqueous phase was extracted with another portion of toluene (750mL). The organic phases were combined and washed with sodium chloride solution (15%, 500mL) and dried over sodium sulphate. The sodium sulphate was filtered off, the filtrate evaporated at reduced pressure to a red oil which was processed further in the next step.

### 4-(2-(4-tolylsurlfanyl)phenyl)-piperidin hydrobromide

The crude ethyl 4-(2-(4-tolylsulfanyl)phenyl)-piperidin-1-carboxylate as a red oil from example 3 was mixed in a stirred reactor with hydrobromic acid in acetic acid (40%, 545mL, 3.11mol). The mixture was heated at 80°C for 18 hours. The reaction mixture was cooled down to room temperature. During the cooling the product crystallises out. After 1 hour at room temperature ethyl ether (800mL) was added to the reaction mixture, and the mixture was stirred for another hour. The product was filtered off, washed with ethyl ether and dried in a vacuum oven at 50°C until constant weight.

### Example 2B 4-[2-(4-methylphenylsulfanyl)phenyl]piperidine, HBr salt

To 442 grams of stirred and slightly heated (approx. 45 °C) 4-(2-p-Tolylsulfanyl-phenyl)-piperidine-1-carboxylic acid ethyl ester as an oil was added 545 ml of 33 wt-% HBr in AcOH (5.7 M, 2.5 eqv.). This mixing gives a 10 °C exotherm. After final addition the reaction mixture is heated to 80 °C and left for 18 hours. A sample is withdrawn and analysed by HPLC and if not completed more 33 wt-% HBr in AcOH must be added. Otherwise the mixture is cooled to 25 °C making the product 4-(2-p-Tolylsulfanyl-phenyl)-piperidine hydrobromide to precipitate. After one hour at 25 °C the thick suspension is added 800 ml diethylether. Stirring is continued for another hour before the product is isolated by filtration, washed with 400 ml diethylether and dried in vacuum at 40 °C overnight. The hydrobromide of compound I was isolated as white solid.

### Example 2C Recrystallisation of 4-[2-(4-methylphenylsulfanyl)phenyl]piperidine, HBr salt

A mixture of 10.0 grams of the HBr salt, e.g. prepared as above, was heated to reflux in 100 ml H₂O. The mixture became clear and fully dissolved at 80-90 °C. To the clear solution was added 1 gram of charcoal and reflux was continued for 15 minutes before filtered and left to cool spontaneously to room temperature. During the cooling precipitation of white solid took place and the suspension was stirred for 1 hour at room temperature. Filtration and drying in vacuum at 40 °C overnight produced 6.9 grams (69 %) of the HBr acid addition salt of 4-[2-(4-methylphenylsulfanyl)phenyl]-piperidine.

### Example 3 4-[2-(4-methylphenylsulfany)phenyl]piperidine, further salts

### Preparation of stock-solutions of free base

A mixture of 500 ml ethyl acetate and 200 ml H₂O was added 50 grams of the HBr salt producing a two-phased slurry. To this slurry was added approximately 25 ml conc. NaOH that caused formation of a clear two-phased solution (pH was measured to 13-14). The solution was stirred vigorously for 15 minutes and the organic phase was separated. The organic phase was washed with 200 ml H₂O, dried over Na₂SO₄, filtered and evaporated in vacuum at 60 °C producing the free base in 38 grams yield (99 %) as an almost colourless oil.

Dissolving 10 grams of the oil and adjusting the volume to 150 ml using ethyl acetate produced a 0.235 M stock-solution in ethyl acetate from which aliquots of 1.5 ml (100 mg of the free base) was used.

Dissolving 10 grams of the oil and adjusting the volume to 100 ml using 96-vol% EtOH produced a 0.353 M stock-solution in EtOH from which aliquots of 1.0 ml (100 mg of the free base) was used.

### Formation of salts using stock-solutions of the free base

The given aliquots were placed in test tubes and while stirred the appropriate amount of acid was added as indicated in Table 1. If the acid was a liquid it was added neat otherwise it was dissolved in the given solvent prior to addition. After mixing and precipitation stirring was continued overnight and the precipitate collected by filtration. Table 2 shows the solubility of salts of 4-[2-(4-methylphenylsulfanyl)-phenyl]piperidine.

**Table 1**

| Acid (Base:Acid) | MW (g/mol) | Amount of Acid (mg or µl) | Solvent | CHN (exp.) | CHN (theory) |
|---|---|---|---|---|---|
| Palmitic acid. hexadecanoic acid 1:1 | 256.42 | 90.5 | EtOAc | 75.36 9.77 2.46 | 75.64 9.9 2.6 |
| DL-Lactic acid, DL-2hydroxypropionic acid 1:1 | 90.1 | 31.8 | EtOAc | 66.88 7.26 3.52 | 67.53 7.29 3.75 |
| Adipicacid, 1,6-hexanedioic acid 1:1 | 146.14 | 51.6 | EtOAc | 66.08 7.23 2.98 | 67.1 7.27 3.26 |
| Adipicacid, 1,6-hexanedioic acid 2:1 | 146.14 | 25.8 | EtOAc | 70.66 7.32 3.82 | 70.75 7.35 3.93 |
| Fumanc acid 1:1 | 116.01 | 40.9 | EtOH | 65.71 6.41 3.35 | 66.14 6.31 3.51 |
| Glutaric acid, 1,5-pentanedioic acid 1:1 | 132.12 | 46.6 | EtOAc | 66.09 6.97 3.2 | 66.48 7.03 3.37 |
| Malonic acid 1:1 | 104.1 | 36.7 | EtOAc | 65.04 6.53 3.54 | 65.09 6.5 3.62 |
| Oxalic acid 1:1 | 90.1 | 31.8 | EtOH | 64.28 6.41 3.61 | 64.32 6.21 3.75 |
| Sebacoinic acid, 1,8-octanedioic acid 2:1 | 202.02 | 35.6 | EtOAc | 71.79 7.86 3.58 | 71.83 7.86 3.64 |
| Succinic acid, 1,4-butanedioic acid, 2:1 | 118.1 | 20.8 | EtOAc | 65.65 6.86 3.4 | 65.80 6.78 3.49 (1:1 salt formed) |
| L-malic acid, L-2-hydroxy butanedioic acid 1:1, α | 134.1 | 47.3 | EtOAc | 62.87 6.20 3.22 | 63.29 6.52 3.36 |
| L-malic acid, L-2-hydroxy butanedioic acid 1:1, β | 134.1 | 47.3 | EtOH | 62.99 6.66 3.13 | 63.29 6.52 3.36 |
| D-tartaric acid, D-2,3-dihydroxy butanedioic acid 1:1 | 150.1 | 53.0 | EtOH | 60.67 6.4 3.07 | 60.95 6.28 3.23 |
| L-aspartic acid 1:1 | 133.1 | 47.0 | EtOH | 59.31 6.7 7.1 (contains excess of acid) | 63.43 6.78 6.73 |
| Glutamic acid 1:1 | 165.15 | 58.3 | EtOH | 56.38 6.88 7.35 (contains excess of acid) | 56.46 6.94 7.06 (for 1:1-salt and acid-monohydrate 1:1) |
| Citric acid 2:1 | 192.13 | 33.9 | EtOAc | 65.93 6.72 3.44 | 66.46 6.64 3.69 |
| HCl/Et₂O 1:1 | 2M | 176.4 | EtOH | | |
| Phosphoric acid 1:1 | 14.7 M | 24.0 | EtOAc | 55.79 6.47 3.43 | 56.68 6.34 3.67 |

**Table 2**

| Acid (Base:Acid) | Solubility (mg/ml) |
|---|---|
| Palmitic acid, hexadecanoic acid 1:1 | 0.4 |
| DL-Lactic acid, DL-2- hydroxypropionic acid 1:1 | >150 |
| Adipicacid, 1,6-hexanedioic acid 1:1 | 2.5 |
| Adipicacid, 1,6-hexanedioic acid 2:1 | 1.0 |
| Fumaric acid 1:1 | 0.2 |
| Glutanc acid, 1,5- pentanedioic acid 1:1 | 13 |
| Malonic acid 1:1 (α) | 5.2 |
| Oxalic acid 1:1 | 1.1 |
| Sebacoinic acid, 1,8-octanedioic acid 2:1 | 0.7 |
| Succinic acid. 1,4-butanedioic acid, 2:1 | 2.0 |
| L-malic acid, L-2-hydroxy butanedioic acid 1:1, β | 2.8 |
| D-tartaric acid, D-2,3-dihydroxy butanedioic acid 1:1 | 1.8 |
| L-aspartic acid 1:1 | 39 |
| Glutamic acid 1:1 | >35 |
| Citric acid 2:1 | 0.5 |
| Phosphoric acid 1:1 | 6.0 |
| HCl | 4.5 |
| HBr | 2.4 |

### Example 4 Effect on acetylcholine levels

The experiment was designed to evaluate the effects of compound I on extracellular levels of acetylcholine in the prefrontal cortex and ventral hippocampus of freely-moving rats.

Male Sprague-Dawley rats, initially weighing 275-300 g, were used. The animals were housed under a 12-hr light/dark cycle under controlled conditions for regular in-door temperature (21±2°C) and humidity (55±5%) with food and tap water available ad libitum.

### Surgery and microdialysis experiments

Rats were anaesthetised with hypnorm/dormicum (2 ml/kg) and intracerebral guide cannulas (CMA/12) were stereotaxically implanted into the hippocampus, aiming to position the dialysis probe tip in the ventral hippocampus (co-ordinates: 5,6 mm posterior to bregma, lateral-5,0 mm, 7,0 mm ventral to dura or in the frontal cortex (co-ordinates: 3,2 mm anterior to bregma; lateral, 0,8 mm; 4,0 mm ventral to dura). Anchor screws and acrylic cement were used for fixation of the guide cannulas. The body temperature of the animals was monitored by rectal probe and maintained at 37°C. The rats were allowed to recover from surgery for 2 days, housed singly in cages. On the day of the experiment a microdialysis probe (CMA/12, 0,5 mm diameter, 3 mm length) was inserted through the guide cannula.

The probes were connected via a dual channel swivel to a microinjection pump. Perfusion of the microdialysis probe with filtered Ringer solution (145 mm NaCl, 3 mM KCl, 1 mM MgCl₂, 1,2 mM CaCl₂ containing 0.5 µM neostigmine) was begun shortly before insertion of the probe into the brain and continued for the duration of the experiment at a constant flow rate of 1 µl/min. After 180 min of stabilisation, the experiments were initiated. Dialysates were collected every 20 min. After the experiments the animals were sacrificed, their brains removed, frozen and sliced for probe placement verification.

### Analysis of dialysate acetylcholine

Concentration of acetylcholine (ACh) in the dialysates was analysed by means of HPLC with electrochemical detection using a mobile phase consisting of 100 mM disodium hydrogenphosphate, 2.0 mM octane sulfonic acid, 0.5 mM tetramethylammonium chloride and 0.005% MB (ESA), pH 8.0. A pre-column enzyme reactor (ESA) containing immobilised choline oxidase eliminated choline from the injected sample (10 µl) prior to separation of ACh on the analytical column (ESA ACH-250); flow rate 0.35 ml/min, temperature: 35°C. After the analytical column the sample passed through a post-column solid phase reactor (ESA) containing immobilised acetylcholineesterase and choline oxidase. The latter reactor converted ACh to choline and subsequently choline to betaine and H₂O₂. The latter was detected electrochemical by using a platinum electrode (Analytical cell: ESA, model 5040).

### Data presentation

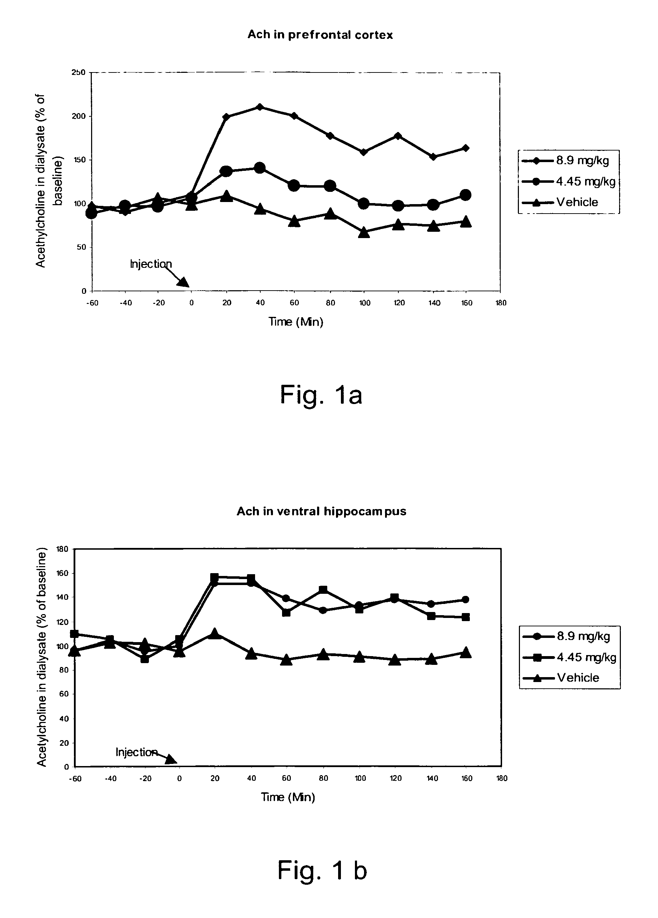

In single injection experiments the mean value of 3 consecutive ACh samples immediately preceding compound administration served as the basal level for each experiment and data were converted to percentage of basal (mean basal pre-injection values normalized to 100%). The data are presented in Figure 1a and 1b.

The data presented in figure 1a and 1b show a dose dependent increase in the extracellular acetylcholine levels in the brain. This pre-clinical finding is expected to translate into an improvement in cognition in a clinical setting useful e.g. in the treatment of cognitive impairment and diseases characterised by a cognitive impairment.

### Example 5 Effect on dopamine levels

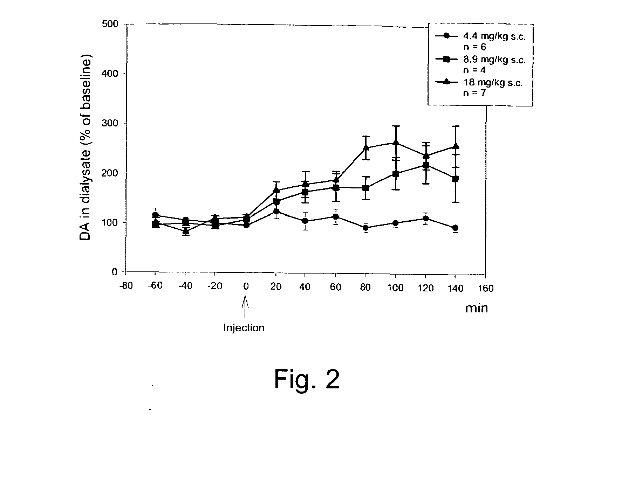

A single injection of compound I dose-dependently increased extracellular dopamine (DA) levels in the rat frontal cortex. The compound of the present invention at 8.9mg/kg and 18mg/kg s.c., enhanced the DA levels by approximately 100% and 150%, respectively, above baseline levels as depicted in figure 2. Amounts are calculated as the free base.

### Method.

Male Sprague-Dawley rats, initially weighing 275-300 g, were used. The animals were housed under a 12-hr light/dark cycle under controlled conditions for regular in-door temperature (21±2°C) and humidity (55±5%) with food and tap water available ad libitum. For the three-day treatment experiments osmotic minipumps (Alzet, 2ML1) were used. The pumps were filled under aseptic conditions and implanted subcutaneously under sevoflurance anaesthesia. The experiments were carried out with the minipumps on board. Blood samples for measuring plasma levels of the test compound after 3 days of treatment were collected at the end of the experiments.

### Surgery and microdialysis experiments.

Animals were anaesthetised with hypnorm/dormicum (2 ml/kg) and intracerebral guide cannulas (CMA/12) were stereotaxically implanted into the hippocampus, positioning the dialysis probe tip in the ventral hippocampus (co-ordinates: 5,6 mm anterior to bregma, lateral-5,0 mm, 7,0 mm ventral to dura or in the frontal cortex (co-ordinates: 3,2 mm anterior to bregma; lateral, 3.0 mm; 4,0 mm ventral to dura). Anchor screws and acrylic cement were sued for fixation of the guide cannulas. The body temperature of the animals was monitored by rectal probe and maintained at 37°C. The rats were allowed to recover from surgery for 2 days, housed singly in cages. On the day of the experiment a microdialysis probe (CMA/12, 0,5 mm diameter, 3 mm length) was inserted through the guide cannula. The probes were connected via a dual channel swivel to a microinjection pump. Perfusion of the microdialysis probe with filtered Ringer solution (145 mm NaCl, 3 mM KCl, 1 mM MgCl₂, 1,2 mM CaCl₂) was begun shortly before insertion of the probe into the brain and continued for the duration of the experiment at a constant flow rate of 1 (1,3) µL/min. After 180 min of stabilisation, the experiments were initiated. Dialysates were collected every 20 (30) min.

After the experiments the rats were sacrificed by decapitation, their brains removed, frozen and sliced for probe placement verification.

### Analysis of dialysates.

Concentration of dopamine in the dialysates was analysed by means of HPLC with electrochemical detection. The monoamines were separated by reverse phase liquid chromatography (ODS 150 x 3 mm, 3 µM). Dopamine: Mobile phase consisting of 90 mM NaH₂PO₄, 50 mM sodium citrate, 367 mg/l sodium 1-octanesulfonic acid, 50 µM EDTA and 8% acetonitrile (pH 4.0) at a flow rate of 0.5 ml/min. Electrochemical detection was accomplished using a coulometric detector; potential set at 250 mV (guard cell at 350 mV) (Coulochem II, ESA).

### Example 6 Effect on neuropatic pain

To demonstrate an efficacy against neuropathic pain, compound I was tested in the formalin model of neuropathic pain [Neuropharm., 48, 252-263, 2005; Pain, 51, 5-17, 1992]. In this model, mice receive an injection of formalin (4.5 %, 20 µl) into the plantar surface of the left hind paw and afterwards are placed into individual glass beakers (2 *l* capacity) for observation. The irritation caused by the formalin injection time spent licking the injured paw. The first phase (~0-10 minutes) represents direct chemical irritation and nociception, whereas the second (~20-30 minutes) is thought to represent pain of neuropathic origin. The two phases are separated by a quiescent period in which behaviour returns to normal. Measuring the amount of time spent licking the injured paw in the two phases assesses the effectiveness of test compounds to reduce the painful stimuli.

Eight C57/B6 mice (ca. 25g) were tested per group. Table 3 below show the amount of time spent licking the injured paw in the two phases, i.e. 0-5 minutes and 20-30 minutes post formalin injection. The amount of compound administered is calculated as the free base.

**Table 3**

| | Vehicle | 1.0 mg/kg | 2.5 mg/kg | 10 mg/kg |
|---|---|---|---|---|
| 0-5 minutes (sec) | 42 | 37 | 30 | 37 |
| 20-30 minutes (sec) | 41 | 43 | 26 | 6 |

The data in table 3 shows that compound I has little effect in the first phase representing direct chemical irritation and nociception. More notably, the data also show a clear and dose dependent decrease in the time spent licking paws in the second phase indicating an effect of compound I in the treatment of neuropathic pain.

## Claims

1. A liquid pharmaceutical formulation comprising a salt of 4-[2-(4-methylphenylsulfanyl)phenyl]piperidine selected from the DL-lactic acid addition salt, the glutaric acid addition salt, the L-aspartic acid addition salt and the glutamic acid addition salt.

2. The liquid formulation according to claim 1 wherein said salt is the DL-lactic acid addition salt.

3. The liquid formulation according to claim 1, wherein said salt is the glutaric acid addition salt.

4. The liquid formulation according to claim 1, wherein said salt is the L-aspartic acid addition salt.

5. The liquid formulation according to claim 1, wherein said slat is the glutamic acid addition salt.

6. The liquid formulation according to any of claims 1-5, wherein the concentration of said salt is above 5 mg/ml.

7. A salt of 4-[2-(4-methylphenylsulfanyl)phenyl]piperidine selected from the DL-lactic acid addition salt, the glutaric acid addition salt, the L-aspartic acid addition salt and the glutamic acid addition salt for use in the treatment of a disease selected from mood disorders, major depressive disorder, general anxiety disorder, atypical depression, bipolar depression, social anxiety disorder, obsessive compulsive disorder, panic disorder, post traumatic stress disorder, abuse, eating disorder, sleep disorder, Alzheimer's disease, dementia, chronic pain, depression associated with cognitive impairment, depression associated with psychosis, cognitive impairment in schizophrenia, depression or anxiety associated with pain, behavioural disturbances in the elderly, ADHD, melancholia, treatment resistant depression or depression with residual symptoms, wherein said salt is in a liquid pharmaceutical formulation.

8. The salt according to claim 7 which salt is the DL-lactic acid addition salt of 4-[2-(4-methylphenylsulfanyl)phenyl]piperidine.

9. The salt according to claim 7, which salt is the glutaric acid addition salt of 4-[2-(4-methylphenylsulfanyl)phenyl]piperidine.

10. The salt according to claim 7, which salt is the L-aspartic acid addition salt of 4-[2-(4-methylphenylsulfanyl)phenyl]piperidine.

11. The salt according to claim 7, which salt is the glutamic acid addition salt of 4-[2-(4-methylphenylsulfanyl)phenyl]piperidine.

12. The salt according to any of claims 7-11, wherein said liquid formulation comprises above 5 mg/ml of said salt.

13. A container fitted with a drop aggregate, which container comprises a liquid formulation according to any of claims 1-6.

## Patentansprüche

1. Flüssige pharmazeutische Formulierung, umfassend ein Salz von 4-[2-(4-Methylphenylsulfanyl)phenyl]piperidin, ausgewählt aus DL-Milchsäure-Additionssalz, Glutarsäure-Additionssalz, L-Asparaginsäure-Additionssalz und Glutaminsäure-Additionssalz.

2. Flüssige Formulierung nach Anspruch 1, worin das Salz das DL-Milchsäure-Additionssalz ist.

3. Flüssige Formulierung nach Anspruch 1, worin das Salz das Glutarsäure-Additionssalz ist.

4. Flüssige Formulierung nach Anspruch 1, worin das Salz das L-Asparaginsäure-Additionssalz ist.

5. Flüssige Formulierung nach Anspruch 1, worin das Salz das Glutaminsäure-Additionssalz ist.

6. Flüssige Formulierung nach einem der Ansprüche 1 bis 5, worin die Konzentration des Salzes oberhalb von 5 mg/ml ist.

7. Salz von 4-[2-(4-Methylphenylsulfanyl)phenyl]piperidin, ausgewählt aus dem DL-Milchsäure-Additionssalz, Glutarsäure-Additionssalz, L-Asparaginsäure-Additionssalz und Glutaminsäure-Additionssalz zur Verwendung in der Behandlung einer Erkrankung, ausgewählt aus Gemütsstörungen, Hauptdepressionsstörung, allgemeiner Angststörung, atypischer Depression, bipolarer Depression, sozialer Angststörung, obsessiv-kompulsiver Störung, Panikstörung, posttraumatischer Stressstörung, Missbrauch, Essstörung, Schlafstörung, Alzheimererkrankung, Demenz, chronischem Schmerz, Depression, assoziiert mit kognitiver Beeinträchtigung, Depression, assoziiert mit Psychose, kognitiver Beeinträchtigung bei der Schizophrenie, Depression oder Angstzustand, assoziiert mit Schmerz, Verhaltensstörungen bei Älteren, ADHD, Melancholie, behandlungsresistenter Depression oder Depression mit restlichen Symptomen, worin das Salz in einer flüssigen pharmazeutischen Formulierung vorliegt.

8. Salz nach Anspruch 7, das das Salz von DL-Milchsäure-Additionssalz von 4-[2-(4-Methylphenylsulfanyl)phenyl]-piperidin ist.

9. Salz nach Anspruch 7, das das Salz von Glutarsäure-Additionssalz von 4-[2-(4-Methylphenylsulfanyl)phenyl]-piperidin ist.

10. Salz nach Anspruch 7, das das Salz von L-Asparaginsäure-Additionssalz von 4-[2-(4-Methylphenylsulfanyl)phenyl]-piperidin ist.

11. Salz nach Anspruch 7, das das Salz von Glutaminsäure-Additionssalz von 4-[2-(4-Methylphenylsulfanyl)phenyl]-piperidin ist.

12. Salz nach einem der Ansprüche 7 bis 11, worin die flüssige Formulierung mehr als 5 mg/ml des Salzes umfasst.

13. Behälter, ausgerüstet mit einem Tropfenaggregat, wobei der Behälter eine flüssige Formulierung nach einem der Ansprüche 1 bis 6 umfasst.

## Revendications

1. Formulation pharmaceutique liquide comprenant un sel de la 4-[2-(4-méthylphénylsulfanyl)phényl]pipéridine choisi parmi le sel d'addition d'acide DL-lactique, le sel d'addition d'acide glutarique, le sel d'addition d'acide L-aspartique et le sel d'addition d'acide glutamique.

2. Formulation liquide selon la revendication 1, dans laquelle ledit sel est le sel d'addition d'acide DL-lactique.

3. Formulation liquide selon la revendication 1, dans laquelle ledit sel est le sel d'addition d'acide glutarique.

4. Formulation liquide selon la revendication 1, dans laquelle ledit sel est le sel d'addition d'acide L-aspartique.

5. Formulation liquide selon la revendication 1, dans laquelle ledit sel est le sel d'addition d'acide glutamique.

6. Formulation liquide selon l'une quelconque des revendications 1 à 5, dans laquelle la concentration dudit sel est supérieure à 5 mg/ml.

7. Sel de la 4-[2-(4-méthylphénylsulfanyl)phényl]pipéridine choisi parmi le sel d'addition d'acide DL-lactique, le sel d'addition d'acide glutarique, le sel d'addition d'acide L-aspartique et le sel d'addition d'acide glutamique pour une utilisation dans le traitement d'une maladie choisie parmi les troubles de l'humeur, un trouble dépressif majeur, un trouble d'anxiété générale, une dépression atypique, une dépression bipolaire, un trouble d'anxiété sociale, un trouble obsessionnel compulsif, un trouble de panique, un trouble de stress post-traumatique, une toxicomanie, un trouble de l'alimentation, un trouble du sommeil, la maladie d'Alzheimer, une démence, une douleur chronique, une dépression associée à une altération de la cognition, une dépression associée à une psychose, l'altération de la cognition dans la schizophrénie, une dépression ou une anxiété associée à une douleur, des troubles comportementaux chez les personnes âgées, le THADA, la mélancolie, une dépression résistante à un traitement ou une dépression avec des symptômes résiduels, où ledit sel est dans une formulation pharmaceutique liquide.

8. Sel selon la revendication 7, lequel sel est le sel d'addition d'acide DL-lactique de la 4-[2-(4-méthylphénylsulfanyl)phényl]pipéridine.

9. Sel selon la revendication 7, lequel sel est le sel d'addition d'acide glutarique de la 4-[2-(4-méthylphénylsulfanyl)phényl]pipéridine.

10. Sel selon la revendication 7, lequel sel est le sel d'addition d'acide L-aspartique de la 4-[2-(4-méthylphénylsulfanyl)phényl]pipéridine.

11. Sel selon la revendication 7, lequel sel est le sel d'addition d'acide glutamique de la 4-[2-(4-méthylphénylsulfanyl)phényl]pipéridine.

12. Sel selon l'une quelconque des revendications 7 à 11, ou ladite formulation liquide comprend plus de 5 mg/ml dudit sel.

13. Récipient équipé d'un dispositif de transfert goutte à goutte, lequel récipient comprend une formulation liquide selon l'une quelconque des revendications 1 à 6.
